# NEW EUROPEAN PATENT SPECIFICATION

(11) **EP 0 758 381 B2**
(45) Date of publication and mention of the opposition decision: **15.11.2006**
(45) Mention of the grant of the patent: 19.02.2003
(21) Application number: 95916204.1
(22) Date of filing: 04.04.1995
(51) Int. Cl.: C07K 14/47

(54) **BIOLOGICALLY ACTIVE EPH FAMILY LIGANDS**
BIOLOGISCH AKTIVE LIGANDE DER EPH FAMILIE
LIGANDS DE LA FAMILLE DES EPH A ACTIVITE BIOLOGIQUE

(30) Priority: 04.04.1994 US 222075; 12.04.1994 US 229402; 01.09.1994 US 299567; 21.10.1994 US 327423
(43) Date of publication of application: 19.02.1997
(73) Proprietor: REGENERON PHARMACEUTICALS, INC., Tarrytown, NY 10591-6707 (US)
(72) Inventor: DAVIS, Samuel, New York, NY 10024 (US); GALE, Nicholas, New Rochelle, NY 10804 (US); ALDRICH, Thomas H., Ossining, NY 10562 (US); MAISONPIERRE, Peter C., Croton, NY 10520 (US); GOLDFARB, Mitchell, River Edge, NJ 07661 (US); YANCOPOULOS, George D., Yorktown Heights, NY 10598 (US)
(74) Representative: Duckworth, Timothy John
(86) International application number: PCT/US1995/004208
(87) International publication number: WO 1995/027060

(56) References cited:
- WO-A1-92/12119
- WO-A1-94/11020
- WO-A1-94/11384
- WO-A1-95/06065
- DAVIS, SAMUEL ET AL.: 'LIGANDS FOR EPH RELATED RECEPTOR TYROSINE KINASES THAT REQUIRE MEMBRANE ATTACHMENT OR CLUSTERING FOR ACTIVITY' SCIENCE vol. 266, no. 5186, pages 816 - 819, XP002915266
- V. LHOTAK AND T. PAWSON: 'BIOLOGICAL AND BIOCHEMICAL ACTIVITIES OF A CHIMERIC EPIDERMAL GROWTH FACTOR-ELK RECEPTOR TYROSINE KINASE' MOLECULAR AND CELLULAR BIOLOGY vol. 13, no. 11, pages 7071 - 7079, XP002988521
- Cell Vol. 90, pages 403-404 "Unified Nomenclature for Eph Family Receptors and their Ligands, the Ephrins

## Description

The present invention provides methods for enhancing the biological activity of soluble forms of ligands that bind proteins belonging to the Eph subfamily of receptorlike protein tyrosine kinases, such as the Ehks (including Ehk-1, Ehk-2 and Ehk-3), Eck, and Elk. It also relates to biologically active, clustered forms of the ligand and to a novel ligand that binds the Ehk-1 receptor.

### BACKGROUND OF THE INVENTION

The ability of polypeptide ligands to bind cells and thereby elicit a phenotypic response such as cell growth, survival or differentiation is often mediated through transmembrane tyrosine kinases. The extracellular portion of each receptor tyrosine kinase (RTK) is generally the most distinctive portion of the molecule, as it provides the protein with its ligand-recognizing characteristic. Binding of a ligand to the extracellular domain results in signal transduction via an intracellular tyrosine kinase catalytic domain which transmits a biological signal to intracellular target proteins. The particular array of sequence motifs of this cytoplasmic, catalytic domain determines its access to potential kinase substrates (Mohammadi, et al., 1990, Mol. Cell. Biol., 11: 5068-5078; Fantl, et al., 1992, Cell, 69:413-413).

RTKs appear to undergo dimerization or some related conformational change following ligand binding (Schlessinger, J., 1988, Trend Biochem. Sci. 13: 443-447; Ullrich and Schlessinger, 1990, Cell, 61: 203-212; Schlessinger and Ullrich, 1992, Neuron 9: 383-391); molecular interactions between dimerizing cytoplasmic domains lead to activation of kinase function. In some instances, such as the growth factor platelet derived growth factor (PDGF), the ligand is a dimer that binds two- receptor molecules (Hart, et al. , 1988, Science, 240: 1529-1531; Heldin, 1989, J. Biol. Chem. 264:8905-8912) while, for example, in the case of EGF, the ligand is a monomer (Weber, et al., 1984, J. Biol. Chem., 259:14631-14636).

The tissue distribution of a particular tyrosine kinase receptor within higher organisms provides relevant data as to the biological function of the receptor. The tyrosine kinase receptors for some growth and differentiation factors, such as fibroblast growth factor (FGF) are widely expressed and therefore appear to play some general role in tissue growth and maintenance. Members of the Trk RTK family (Glass & Yanco-poulos, 1993, Trends in Cell Biol, 3:262-268) of receptors are more generally limited to cells of the nervous system, and the Nerve Growth Factor family consisting of NGF, BDNF, NT-3 and NT-4/5 (known as the neurotrophins) which bind these receptors promote the differentiation of diverse groups of neurons in the brain and periphery (Lindsay, R. M, 1993, in Neurotrophic Factors, S.E. Loughlin & J.H. Fallon, eds., pp. 257-284 (San Diego, CA: Academic Press). The localization of one such Trk family receptor, trkB, in tissue provided some insight into the potential biological role of this receptor, as well as the ligands that bind this receptor (referred to herein as cognates). Thus, for example, in adult mice, trkB was found to be preferentially expressed in brain tissue, although significant levels of trkB mRNAs were also observed in lung, muscle, and ovaries. Further, trkB transcripts were detected in mid and late gestation embryos. In situ hybridization analysis of 14 and 18 day old mouse embryos indicated that trkB transcripts were localized in the central and peripheral nervous systems, including brain, spinal cord, spinal and cranial ganglia, paravertebral trunk of the sympathetic nervous system and various innervation pathways, suggesting that the trkB gene product may be a receptor involved in neurogenesis and early neural development as well as play a role in the adult nervous system.

The cellular environment in which an RTK is expressed may influence the biological response exhibited upon binding of a ligand to the receptor. Thus, for example, when a neuronal cell expressing a Trk receptor is exposed to a neurotrophin which binds that receptor, neuronal survival and differentiation results. When the same receptor is expressed by a fibroblast, exposure to the neurotrophin results in proliferation of the fibroblast (Glass, et al., 1991, Cell 66:405-413). Thus, it appears that the extracellular domain provides the determining factor as to the ligand specificity, and once signal transduction is initiated the cellular environment will determine the phenotypic outcome of that signal transduction.

A number of RTK families have been identified based on sequence homologies in their intracellular domain. The receptor and signal transduction pathways utilized by NGF involves the product of the trk proto-oncogene (Kaplan et al., 1991, Nature 350:156-160; Klein et al., 1991, Cell 65:189-197). Klein et al. (1989, EMBO J. 8:3701-3709) reported the isolation of trkB, which encodes a second member of the tyrosine protein kinase family of receptors found to be highly related to the human trk protooncogene. TrkB binds and mediates the functional responses to BDNF, NT-4, and, to a lesser extent, NT-3 (Squinto, et al., 1991, Cell 65:885-903; Ip, et al., 1992, Proc. Natl. Acad. Sci. U.S.A. 89:3060-3064; Klein, et al., 1992, Neuron, 8:947-956). At the amino acid level, the products of trk and trkB were found to share 57 percent homology in their extracellular regions, including 9 of the 11 cysteines present in trk. This homology was found to increase to 88 percent within their respective tyrosine kinase catalytic domains. The Trk gene family has now been expanded to include the trkC locus, with NT-3 having been identified as the preferred ligand for trkC (Lamballe, et al., 1991, Cell 66: 967-979; Valenzuela, et al. 1993, Neuron 10:963-974).

The Eph-related transmembrane tyrosine kinases comprise the largest known family of receptor-like tyrosine kinases, with many members displaying specific expression in the developing and adult nervous system. Two novel members of the Eph RTK family, termed Ehk (eph homology kinase) -1 and -2 were identified using a polymerase chain reaction (PCR)-based screen of genes expressed in brain (Maisonpierre, et al. 1993, Oncogene 8:3277-388). These genes appear to be expressed exclusively in the nervous system, with Ehk-1 expression beginning early in neural development. Recently, a new member of this group of related receptors, Ehk-3 has been cloned (Valenzuela, et al. In press).

The elk gene encodes a receptorlike protein-tyrosine kinase that also belongs to the eph subfamily, and which is expressed almost exclusively in the brain (and at lower levels in the testes) (Letwin, et al. 1988; Oncogene 3:621-678; Lhotak, et al., 1991 Mol. Cell. Biol. 11: 2496-2502. Based on its expression profile, the Elk receptor and its cognate ligand are expected to play a role in cell to cell interactions in the nervous system.

Unlike the Ehks and Elk receptors, the closely related Eck receptor appears to function in a more plei-otropic manner; it has been identified in neural, epithelial and skeletal tissues and it appears to be involved in the gastrulation, craniofacial, and limb bud sites of pattern formation in the mouse embryo (Ganju, et al. 1994, Oncogene 9:1613-1624).

The identification of a large number of receptor tyrosine kinases has far exceeded the identification of their cognate ligands. At best, determination of the tissues in which such receptors are expressed provides insight into the regulation of the growth, proliferation and regeneration of cells in target tissues. Because RTKs appearto mediate a number of important functions during development, their cognate ligands will inevitably play a crucial role in development.

We have found a number of orphan tyrosine kinase receptor-like molecules, including five such molecules that are homologous to trk receptor and the insulin receptor family, and another four molecules that are homologous to, respectively, CSF1 R/PDGFR/kit; ret; eck-alpha (now known as Ehk-2) ; and eck beta (Ehk-1) are described. In that, methods were described wherein cell lines expressing Ehk-1 and Ehk-2 were used to assay for identifying their cognate ligands. Because the ehks appear to be expressed in distinctive neuronal populations, including some of the principal ascending central cholinergic nuclei, ligands which bind these receptors were expected to play a role in promoting the growth or survival of these neuronal cells. Because the expression of Ehk-1 begins early in development, its cognate ligands may play a role in embryogenesis.

Although a number of schemes have been devised for the identification of cognate ligands for the many orphan receptors that have been identified, very few such ligands have been identified, and the ligands that have been identified to date appear to have no activity other than the ability to bind their cognate receptor. For example, International Publication Number WO/94/11020 published on May 26, 1994 describes ligands that bind to the Eck receptor. In particular the ligand EBP (also known as B61) is described. However, although binding of B61 to the Eck receptor is disclosed, no biological activity is described. Similarly, despite the description in PCT Publication Number WO 94/11384 (published May 26, 1994) of a ligand that binds the Elk receptor, no biological activity was observed, regardless of whether the ligand was presented as membrane bound or in the form of an Fc dimer of the soluble ligand. With respect to the Elk receptor, however, chimeric EGFR-Elk receptors (having the extracellular domain of the EGFR fused to the Elk cytoplasmic domain) have been used to demonstrate the functional integrity (as measured by EGF-stimulated autophosphorylation) of the enzymatic domain of this receptor. (Lhotak and Pawson, 1993, Mol. Cell. Biol. 13:7071-7079).

### SUMMARY OF THE INVENTION

According to the invention, soluble forms of the ligands described herein may be used to promote biological responses in Ehk-1, Ehk-2, Ehk-3, Eck and Elk receptor-expressing cells. In particular, a general method is described herein which produces "clustering" of ligands for eph-related receptors, which functions to make otherwise inactive soluble ligands biologically active, orwhich enhances the biological activity of ligands that, absent such clustering, would have only low levels of biological activity.

In one embodiment, the invention provides a method of enhancing the biological activity of the extracellular domain of an eph family ligand comprising:
a) expressing the soluble domain of the ligand with an epitope tag;
b) exposing said tagged soluble domain to anti-tag antibodies to form a clustered eph family ligand,
wherein said biological activity is measured by phosphorylation of the tyrosine kinase domain of an eph family receptor.

The invention also provides an anti-epitope antibody treated preparation of clustered epitope-tagged soluble eph family ligand which is obtainable by such a method wherein the eph family ligand is Efl-1 (B61).

In a further embodiment, the invention provides a method of enhancing the biological activity of the soluble domain of an eph family ligand selected from Efl-1 (B61) comprising:
a) expressing the soluble domain of the ligand with the Fc domain of IgG; and
b) allowing Fc dimers to form wherein said biological activity is measured by phosphorylation of the tyrosine kinase domain of an eph family receptor.

The invention also provides a biologically active, clustered soluble eph family ligand comprising (soluble Efl)ₙ, wherein the soluble Efl is the extracellular domain of Efl-1 (B61) and n is 2 or greater wherein said biological activity is measured by phosphorylation of the tyrosine kinase domain of an eph family receptor.

### DESCRIPTION OF THE FIGURES

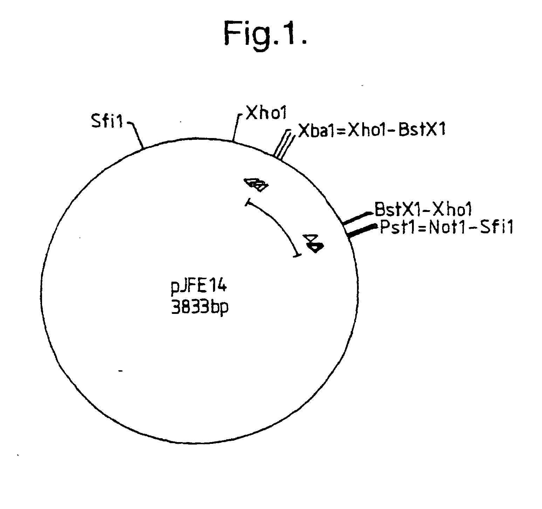

Figure 1. Vector pJFE14.

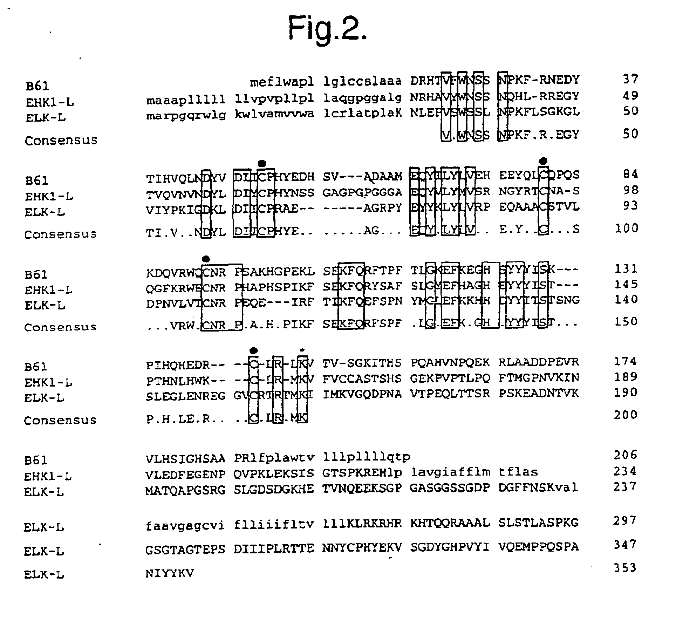

Figure 2. Sequence comparison of ligands for Ehk-1 and Elk receptors. Aligned sequences of B61 (SEQ ID NO:1), Ehk-1 ligand (EHK-1L) (SEQ ID NO:2) and Elk ligand (ELK-L) (SEQ ID NO:3) are displayed. Residues shared by all three sequences are boxed, and residues shared by at least two are shown in the Consensus lane; bold dots indicate conserved cysteines, and asterisks demarcate residues bordering main conserved regions. Lower case letters show the presumed amino-terminal signal sequences as well as the transmembrane domain of ELK-L and carboxy-terminat hydrophobic GPI-recognition tails of B61 and EHK1-L

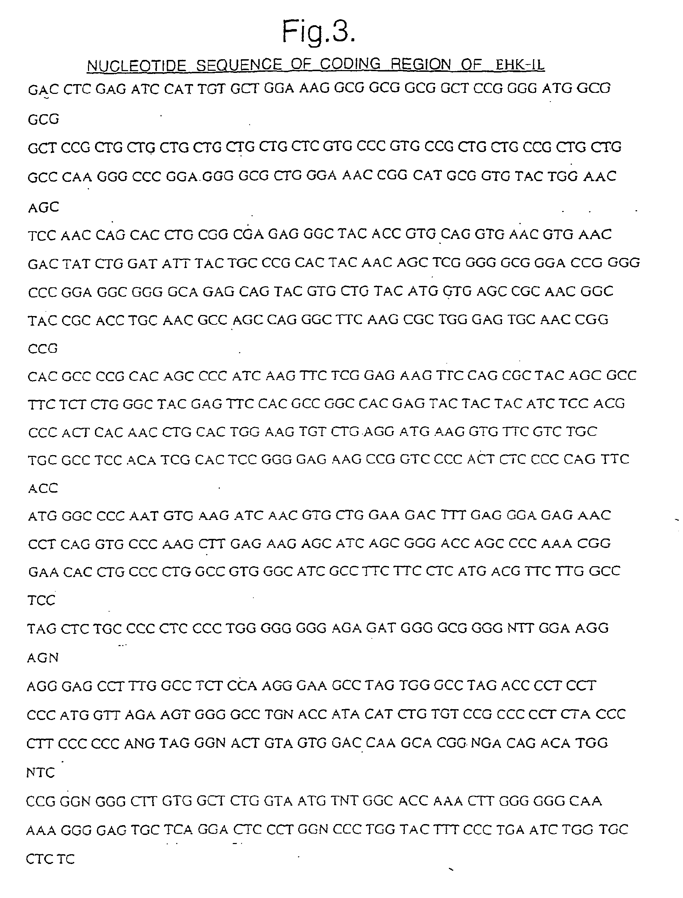

Figure 3. Nucleotide sequence of EHK-1L coding region. (SEQ ID NO: 4).

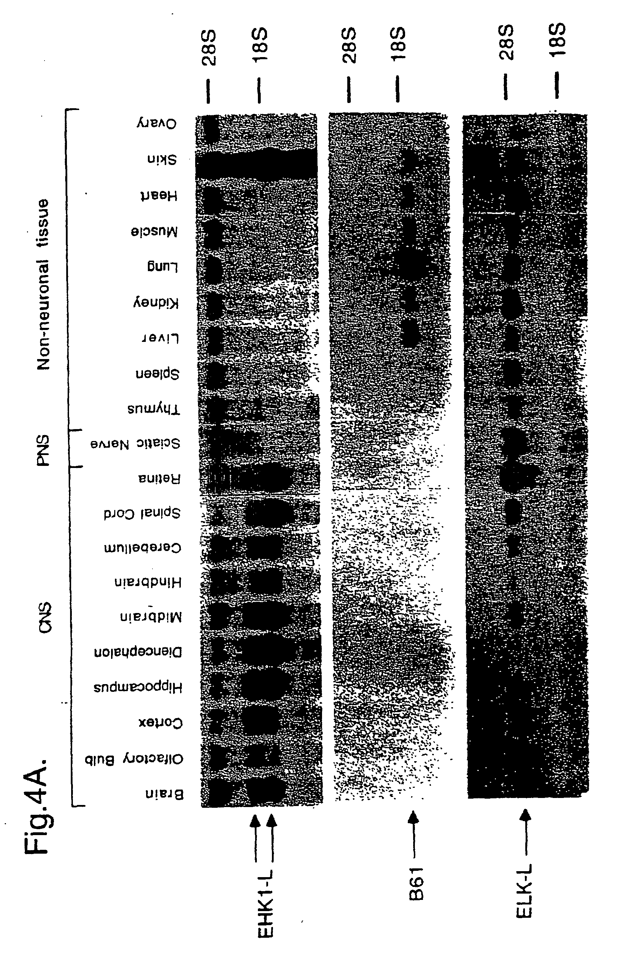

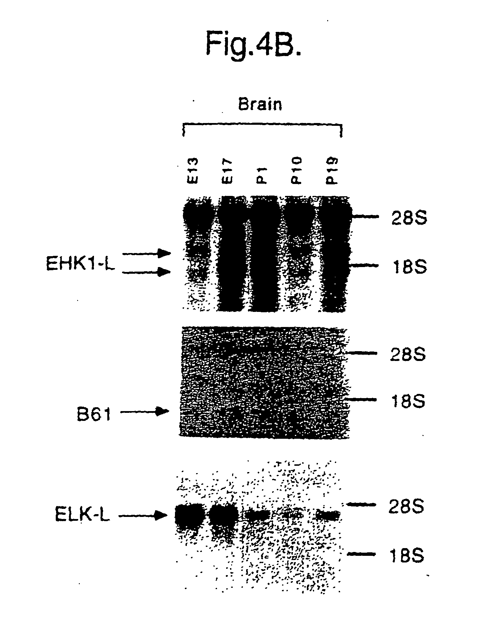

Figure 4. Northern blot analysis of Efl-1 (B61), Efl-2 (EHK-1L) and Efl-3 (ELK-L) expression in adult rat tissues (A) and in developing brain (B). Total RNA (20 micrograms per lane) was isolated from the indicated tissues, fractionated on 1 percent formaldehyde-agarose gels and transferred to nylon membranes. Blots were hybridized to ³²P-labelled probes derived from restriction fragments internal to the coding regions of each cDNA.

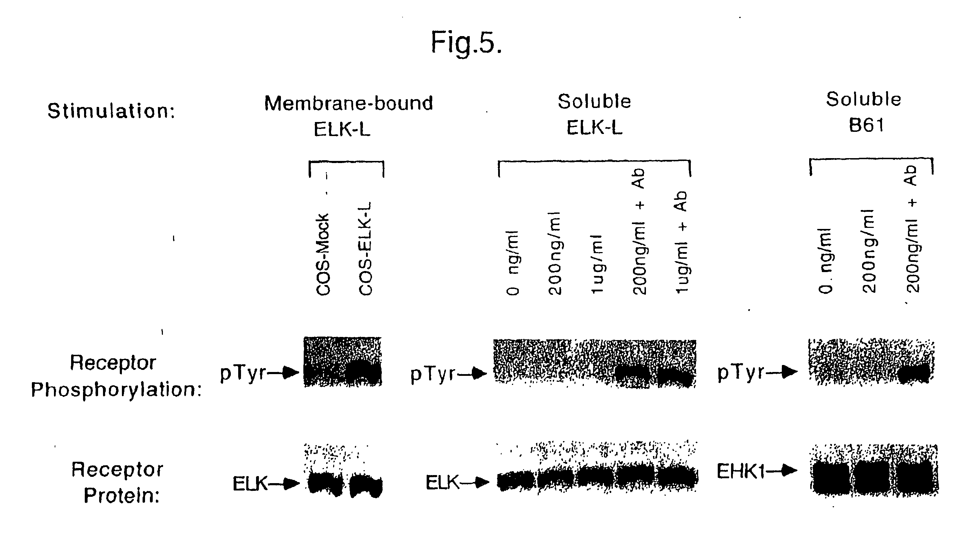

Figure 5. Activation of Eph family receptors by membrane-bound or clustered ligands as measured by Elk receptor tyrosine phosphorylation. Figure 5A: Stimulation using control COS cells transfected with vector alone (COS-Mock) or COS cells transfected with an expression construct encoding Efl-3 (ELK-L); Figure 58: Stimulation with soluble myc-tagged Efl-3 (ELK-L), used as an unclustered ligand (second and third wells) or ligand clustered by antibodies (forth and fifth wells). ("+Ab" denotes soluble ligands clustered with antibody). Control (0 ng/ml) is shown in first well. Figure 5C: Stimulation with soluble myc-tagged Efl-1 (B61), used as an unclustered ligand (second well) or ligand clustered by antibodies (third well). Control (0 ng/ml) is shown in first well. Upper panels in Figures A, B and C show immunoprecipitates immunoblotted with antibodies to phosphotyrosine. Lower panels in Figures A, B and C show immunoprecipitates that were subsequently stripped and reprobed with antibodies recognizing the receptors to visualize total receptor protein.

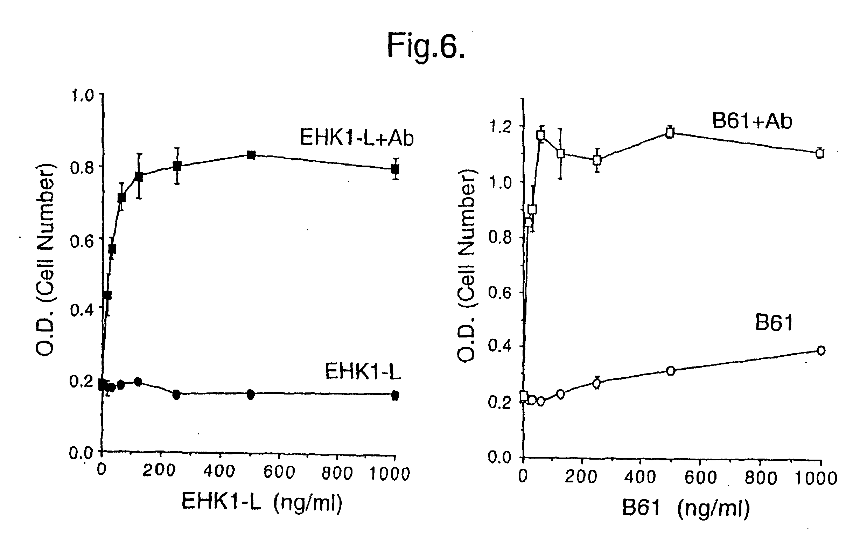

Figure 6. Induction of growth responses in BAF cells mediated by Eck receptor chimera: A. Stimulations were with soluble Efl-2 (EHK-1L) produced in COS cells with or without subsequent clustering. B. Stimulations were with soluble Efl-1 (B61) produced in COS cells with or without subsequent clustering.

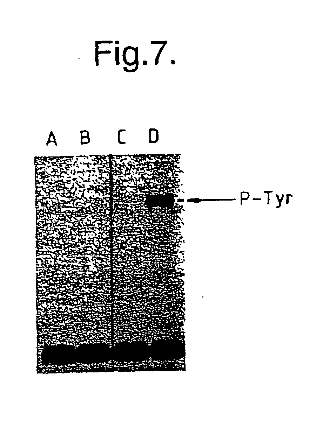

Figure 7. Induction of ELK receptor tyrosine phosphorylation by clustered but not dimeric ligand. Lane A: mock COS supematants without anti-human antibodies; Lane B: anti-human antibodies; Lane C: ELK-L-Fc without crosslinking antibodies; and Lane D: crosslinking antibodies. Soluble Fc-tagged ELK ligand (ELK-L-Fc) was produced as COS cell supernatant and used as unclustered dimeric ligand, or ligand clustered by antibodies. Ligand concentrations were estimated by quantitating the amount of Fc epitope using an elisa assay. Ligand clustering was accomplished by adding anti-Human antibodies and Incubating at room temperature for 1 hour before stimulating cells, Reporter cells were NIH 3T3 fibroblasts transfected with ELK. Cells were starved for 4-6 hours in serum free medium, and then stimulated for 40 minutes with: Following stimulation, cells were solubilized and immunoprecipitated with antibodies recognizing the receptors. Immunoprecipitates were immunoblotted with antibodies to phosphotyrosine.

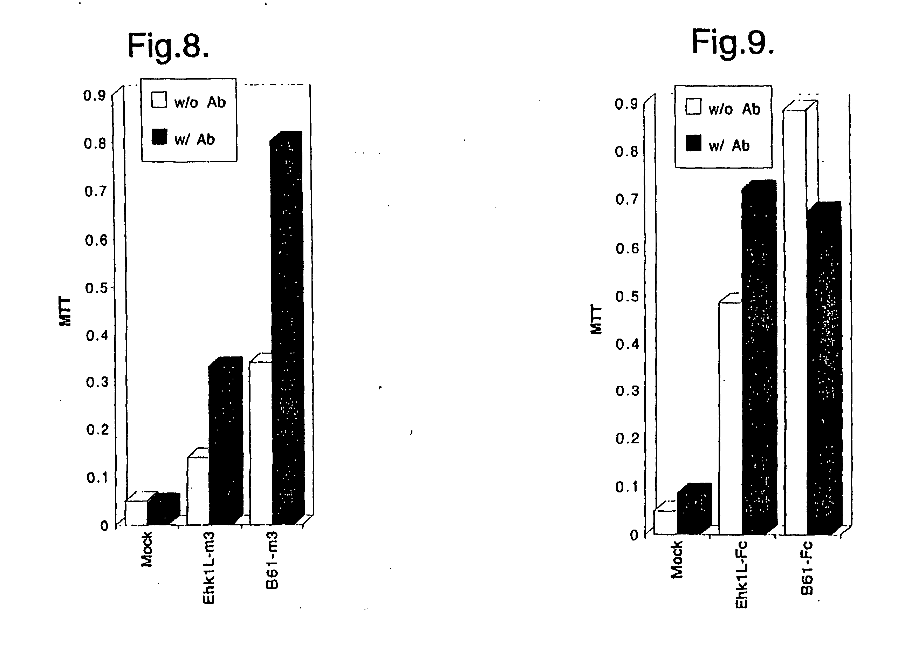

Figure 8. Induction of growth responses in BAF cells mediated by ECK receptor chimera. Stimulations were performed with soluble triple myc-tagged EHK1 ligand (EHK1L-m3) and triple myc-tagged B61 (B61-m3) produced in COS cells with or without subsequent clustering with anti-myc and anti-mouse antibodies. Because the catalytic domain of ECK receptor does not mediate growth response in BAF cells, a receptor chimera (in which the ectodomain of the ECK receptor was fused to the cytoplasmic domain of the FGF receptor) was instead introduced into BAF cells to make the reporter cell line.
Figure 9. Induction of growth responses in BAF cells mediated by ECK receptor chimera. Stimulations were performed with soluble EHK1-L-Fc and B61-Fc produced in COS cells with or without subsequent clustering with anti-human antibodies.

### DETAILED DESCRIPTION OF THE INVENTION

The utilization of assay systems utilizing the Ehk-1, Ehk-2, Ehk-3, Eck and Elk receptors has led to the discovery of cognate ligands to these receptors. Such ligands are useful for promoting the growth and survival of neuronal, epithelial, or other receptor-bearing cell populations *in vitro.*

Applicants have discovered that the previously identified ligand B61 (Holzman, *et al*., 1990, Mol. Cell. Biol. 10:5830-5838) also binds to Ehk-1 and that both B61 and the Ehk-1 ligands also bind the Ehk-2 and Ehk-3 receptor, as well as the Eck receptor.

The ligands described herein are designated as Efl's (Eph transmembrane tyrosine kinase family ligands). The amino acid sequences of B61 (Efl-1) and EHK-1 L (Efl-2), as well as the sequence of the Elk binding ligand Efl-3, which was described in WO 94/1102D (published on May 26, 1994) are set forth in Figure 2. An additional ligand that binds Ehk-1, Ehk-2 and Ehk-3, as well as the Eck receptor, has been identified as Efl-4. Plasmid pJFE14 encoding the ligand Efl-4 was deposited under the terms of the Budapest Treaty on October 21, 1994 as 75921.

When used herein, Efl-1, Efl-2, Efl-3 and Efl-4 include functionally equivalent molecules in which amino acid residues are substituted for residues within the sequence resulting in a silent change. For example, one or more amino acid residues within the sequence can be substituted by another amino acid of a similar polarity which acts as a functional equivalent, resulting in a silent alteration. Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid.

Cells that express Efl's may do so naturally or may be genetically engineered to produce these ligands, as described supra, by transfection, transduction, electroporation, microinjection, via a transgenic animal, etc. of nucleic acid encoding the Efls described herein in a suitable expression vector. Vectors containing the cDNA encoding for EFI-2 (EHK-1 L) were deposited with the American Type Culture Collection under the terms of the Budapest Treaty on April 4, 1994 as pJFE14 containing Ehk-1 L2B4 and Ehk-1L3B1 and have been given the ATCC designations 75728 and 75729 respectively. (The ligands encoded by these vectors are identical). The pJFE14 vector containing the cDNA encoding for Efl-3 (ELK-L) was deposited with the American Type Culture Collection under the terms of the Budapest Treaty-on April 12,1994 as pJFE14 containing *efl*-*3* and has been given the ATCC designation 75734. The pJFE14 vector containing the cDNA encoding for Efl-4 was deposited with the American Type Culture Collection under the terms of the Budapest Treaty on October 21,1994 as pJFE14 encoding Efl-4 and has been given the ATCC designation 75921.

In addition, the present invention contemplates use of the ligands described herein in soluble forms, and tagged forms.

Any of the methods known to one skilled in the art for the insertion of DNA fragments into a vector may be used to construct expression vectors encoding Efl's using appropriate transcriptional/translational control signals and the protein coding sequences. These methods may include in vitro recombinant DNA and synthetic techniques and in vivo recombinations (genetic recombination). Expression of nucleic acid sequence encoding the Efl's or peptide fragments thereof may be regulated by a second nucleic acid sequence so that the protein or peptide is expressed in a host transformed with the recombinant DNA molecule. For example, expression of the Efl's described herein may be controlled by any promoter/enhancer element known in the art. Promoters which may be used to control expression of the ligands include, but are not limited to the long terminal repeat as described in Squinto et al., (1991, Cell 65: 1-20); the SV40 early promoter region (Bernoist and Chambon, 1981, Nature 290:304-310), the CMV promoter, the M-MuLV 5' terminal repeat the promoter contained in the 3' long terminal repeat of Rous sarcoma virus (Yamamoto, et al.,1980, Cell 22:787-797), the herpes thymidine kinase promoter (Wagner et al., 1981, Proc. Natl. Acad. Sci. U.S.A. 78:144-1445), the regulatory sequences of the metallothioein gene (Brinster et al., 1982, Nature 296:39-42); prokaryotic expression vectors such as the β-lactamase promoter (Villa-Kamaroff, et al., 1978, Proc. Natl. Acad. Sci. U.S.A. 75: 3727-3731), or the tac promoter (DeBoer, et al., 1983, Proc. Natl. Acad. Sci. U.S.A. 80:21-25), see also "Useful proteins from recombinant bacteria" in Scientific American, 1980, 242:74-94; promoter elements from yeast or otherfungi such as the Gal 4 promoter, the ADH (alcohol dehydrogenase) promoter, PGK (phosphoglycerol kinase) promoter, alkaline phophatase promoter, and the following animal transcriptional control regions, which exhibit tissue specificity and have been utilized in transgenic animals: elastase I gene control region which is active in pancreatic acinar cells (Swift et al., 1984, Cell 38:639-646; Ornitz et al., 1986, Cold Spring Harbor Symp. Quart. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538: Alexander et al., 1987, Mol. Cell. Biol. 7: 1436-1444), mouse mammary tumor virus control region which is active in testicular, breast, lymphoid and mast cells (Leder et al., 1986, Cell 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5: 1639-1648; Hammer et al., 1987, Science 235:53-58); alpha 1-antitrypsin gene control region which is active in the liver (Kelsey et al, 1987, Genes and Devel. 1:161-171), beta-globin gene control region which is active in myeloid cells (Mogram et al., 1985, Nature 315:338-340; Kollias et al., 1986, Cell 46:89-94); myelin basic protein gene control region which is active in oligodendrocyte cells in the brain (Readhead et al., 1987, Cell 48:703-712); myosin light chain-2 gene control region which is active in skeletal muscle (Shani, 1985, Nature 314:283-286), and gonadotropic releasing hormone gene control region which is active in the hypothalamus (Mason et al., 1986, Science 234:1372-1378).

Thus, expression vectors capable of being replicated in a bacterial or eukaryotic host comprising Efl-encoding nucleic acid as described herein, may be used to transfect the host and thereby direct expression of such nucleic acid to produce the Efl proteins, which may then be recovered in biologically active form. As used herein, a biologically active form includes a form capable of binding to the relevant receptor, such as Ehk-1 or Elk, and causing a differentiated function and/or influencing the phenotype of the cell expressing the receptor. Such biologically active forms would, for example, induce phosphorylation of the tyrosine kinase domain of the Ehk-1 or Elk receptor, or stimulation of synthesis of cellular DNA.

Expression vectors containing the gene inserts can be identified by three general approaches: (a) DNA-DNA hybridization, (b) presence or absence of "marker", gene functions, and (c) expression of inserted sequences. In the first approach, the presence of a foreign gene inserted in an expression vector can be detected by DNA-DNA hybridization using probes comprising sequences that are homologous to an inserted *efl* gene. In the second approach, the recombinant vector/host system can be identified and selected based upon the presence or absence of certain "marker" gene functions (e.g., thymidine kinase activity, resistance to antibiotics, transformation phenotype, occlusion body formation in baculovirus, etc.) caused by the insertion of foreign genes in the vector. For example, if the *efl* gene is inserted within the marker gene sequence of the vector, recombinants, containing the insert can be identified by the absence of the marker gene function. In the third approach, recombinant expression vectors can be identified by assaying the foreign gene product expressed by the recombinant. Such assays can be based, for example, on the physical or functional properties of the *efl* gene product, for example, by binding of the ligand to the Ehk-1 or Elk receptor or portion thereof which may be tagged with, for example, a detectable antibody or portion thereof or binding to antibodies produced against the Efl protein or a portion thereof.

The Efl-2 (EHK-1L) ligand appears to have some homology with the previously identified ligand B61 (referred to herein as Efl-1). [Holzman, et al., 1990, Mol. Cell. Biol. 10:5830-5838). Like B61, Efl-2 ends in a C-terminal hydrophobic sequence that appears to encode a recognition sequence allowing it to be GPI-linked and thus lacking in an intracellular domain. In fact, pretreatment of Efl-2 expressing cells with phospholipase C appears to reduce Ehk-1 receptorbody binding, thus confirming that these proteins are Pl-linked. Efl-3 is not cleaved from the cell surface using phospholipase C and it appears to comprise a conventional transmembrane protein with a cytoplasmic domain.

The ligands described herein may be produced as membrane bound forms in animal cell expression systems or may be expressed in soluble form. Soluble forms of the ligands may be expressed using methods known tothose in the art. A commonly used strategy involves use of oligonucleotide primers, one of which spans the N-terminus of the protein, the other of which spans the region just upstream to a hydrophobic segment of the protein, which represents either the GPI-linkage recognition domain or a transmembrane domain of the protein. The oligonucleotide spanning the C-terminus region is modified so as to contain a stop codon prior to the hydrophobic domain. The two oligonucleotides are used to amplify a modified version of the gene encoding a protein that is secreted instead of membrane bound. Alternatively, a convenient restriction site in the vector can be used to insert an altered sequence that removes the GPI-linkage recognition domain or transmembrane domain, thus resulting In a vector capable of expressing a secreted form of the protein. The soluble protein so produced would include the region of the protein from.the N-terminus to the region preceding the hydrophobic GPI recognition domain or transmembrane domain.

Applicants have discovered that although the soluble ligands produced according to the invention bind to the receptors in the eph subfamily, such soluble ligands often have little or-no biological activity. Such soluble ligands are activated, according to the present invention, by ligand "clustering". "Clustering" as used herein refers to any method known to one skilled In the art for creating multimers of the soluble portions of ligands described herein.

In one embodiment, a "clustered" efl is a dimer, made for example, according to the present invention utilizing the Fc domain of IgG (Aruffo et al., 1991, Cell 67:35-44), which results in the expression of the soluble ligand as a disulfide-linked homodimer. In another embodiment, secreted forms of the ligands are constructed with epitope tags at their C-termini; anti-tag antibodies are then used to aggregate the ligands.

Alternatively, multimers may be made by genetically engineering and expressing molecules that consist of the soluble or extracellular portion of the ligand followed by the Fc-domain of hlgG, followed by either the c-jun or the c-fos leucine zippers described above [Kostelny et al., J. lmmunol, 148: 1547-1553 (1992)]. Since these leucine zippers form predominately heterodimers, they may be used to drive formation of the heterodimers where desired. As for the chimeric proteins described using leucine zippers, these may also be tagged with metal chelates or an epitope. This tagged domain can be used for rapid purification by metal-chelate chromatography, and/or by antibodies, to allow for detection on western blots, immunoprecipitation, or activity depletion/blocking in bioassays.

Multimeric soluble ligands may be prepared by expression as chimeric molecules utilizing flexible linker loops. A DNA construct encoding the chimeric protein Is designed such that it expresses two or more soluble or extracellular domains fused together in tandem ("head to head") by a flexible loop. This loop may be entirely artificial (e. g. polyglycine repeats interrupted by serine or threonine at a certain interval) or "borrowed" from naturally occurring proteins (e.g. the hinge region of hlgG). Molecules may be engineered in which the length and composition of the loop is varied, to allow for selection of molecules with desired characteristics. Although not wishing to be bound by theory, applicants believe that membrane attachment of the ligands facilitates ligand clustering, which in turn promotes receptor multimerization and activation. Thus, according to the invention, biological activity of the soluble ligand is achieved by mimicking, in solution, membrane associated ligand clustering. Thus, a biologically active, clustered soluble eph family ligand comprises (soluble Efl)ₙ, wherein the soluble efl is the extracellular domain of ligand that binds an eph family receptor and n is 2 or greater. As described herein, Efl-1, Efl-2 and Efl-3 are all made biologically active according the the process of the invention.

In each case, one skilled in the art will recognize that the success of clustering will require analysis of the biological activity utilizing bioassays such as those described herein. For example, as shown in Fig. 5, despite the fact that receptor phosphorylation is markedly induced by stimulating receptor expressing reporter cells with COS cells overexpressing membrane forms of the ligands B61, Efl-2 and Efl-3 (Fig. 5A, lanes 1 and 2), there is no observable phosphorylation using soluble forms of these ligands (Fig. 5B, lanes 2 and 3, and Figure 5C, lane 2). However, when secreted forms of the ligands are myc-tagged and antibodies are used to cluster the ligands, the previously inactive soluble ligands strongly induce receptortyrosine phosphorylation in reporter cells expressing Elk and Ehk-1 receptors (Figure 5B, lanes 4 and 5, Figure 5C, lane 3) as well as proliferation in reporter cells expressing an Eck receptor chimera (Fig. 6A).

Although in some instances dimerization of the ligand is sufficient to induce biological activity, the data set forth in Figure 7 demonstrates how, in certain instances, the methods described herein are used to determine the sufficiency of a particular clustering technique. As shown in Figure 7 with the Elk ligand, dimerization of the soluble ligand utilizing Fc appears to be insufficient for achieving a biological response (Figure 7, Lane C). Yet, further clustering of the ligand according to the invention using anti-Fc antibodies resulted in a substantial increase In biological activity (lane D).

In the case of the ligand B61, a low level of biological activity appears to be obtained with the soluble ligand achieved without clustering; such low level biological activity may be caused by low level "self-clustering". Regardless, applicants have demonstrated that even in the case of efl's such as B61 that have some biological activity, the activity may be enhanced by clustering according to the present invention.

Cells may transiently or, preferably, constitutively and permanently express the Efl's In native form,or in soluble form as tagged Efl's or clustered Efl's as described herein.

The recombinant factors may be purified by any technique which allows for the subsequent formation of a stable, biologically active protein. For example, and not by way of limitation, the factors may be recovered from cells either as soluble proteins or as inclusion bodies, from which they may be extracted quantitatively by 8M guanidinium hydrochloride and dialysis. In order to further purify the factors, conventional ion exchange chromatography, hydrophobic interaction chromatography, reverse phase chromatography or gel filtration may be used.

The potential therapeutic use of cognate ligands to Ehk-1 has been suggested previously. In adult brain, cellular localisations of *ehk*-1 Indicated that the gene is primarily expressed in neurons, and reach their highest levels of expression in distinctive neuronal populations that include some of the principal ascending central cholinergic nuclei (interpeduncular region, olfactory tubercle and lateral amygdala) and monoaminergic nuclei (locus coeruleus, dorsal raphe and substantia nigra). Ehk-1 probes strongly highlighted the piriform cortex in the ventrolateral region of the forebrain, the horizontal limb of the diagonal band, the rostral septum and neurons associated with the indusium griseum and tenia tecta, in the hippocampus, in pyramidal neurons in CA3 and CA2, and somewhat weaker in CA1, In the superior colliculus and in retrosplenial cortex and neurons of the locus coeruleus.

In adult rat brain, Ehk-1 can be seen to strongly highlight neuronal rich densities associated with the interpeduncular nuclei and substantia nigra, the basolateral and lateral amygdala and dorsal raphe. Ehk-1 probes were capable of significant hybridization to the granular layer of the cerebellum, in Purkinje cells in the cerebellum and mitral cells in the olfactory bulb.

In E13 embryos, ehk-1 transcripts are noticeably more abundant in head than in body. By post-natal day 1 (P1) the bands reach their highest level in brain, declining slightly in adult brain. Ehk-1 bands decline in cerebellar samples during the transition from P1 to adults, suggesting that the major sites of expression in adult whole brain are predominantly outside the cerebellum.

Longer exposures demonstrate that the ehk-1 neural-specific bands are also detectable In RNAs from primary cultures of hippocampal astrocytes. This Is unexpected because RNA In situ hybridization studies indicate that the ehk gene is predominantly expression in neurons, not glia. Examination in various established cell lines has Indicated that *ehk*-*1* is predominantly expressed In neuronally derived cells, including various neuroepitheliomal and neuroblastomal lines.

The Elk receptor is also expressed primarily in brain. Accordingly, ft is believed that the Elk binding ligand described herein will support the induction of a differential function and/or influence the phenotype, such as growth and/or survival of neural cells, expressing this receptor.

Northern blot analysis (Figure 4) revealed restricted and reciprocal patterns of expression for Efl-2 (EHK-1 L) and Efl-1 (B61), in contrast to broad distribution of Efl-3 (ELK-L). Like Ehk-1, Efl-2 is expressed almost exclusively in the central nervous system, with the notable exception of high expression in skin, where Ehk-1 expression is almost undetectable. In contrast, Efl-1 (B61) Is expressed primarily in non-neuronal tissues (Fig. 4A; longer exposures reveal low levels of B61 in most neural structures). Unlike the Elk receptor, which is expressed in only the brain and testes, Efl-3 (ELK-L) is widely expressed in both neuronal and non-neuronal tissues. In brain, expression of both Efl-1 and Efl-3, but not Efl-2, is substantially higher early in development and then decreases (Figure 4B). As with Efl-1 and Efl-3, the ligands for other nervous-system specific receptor tyrosine kinases (such as the neurotrophin ligands for the Trk receptors) are also expressed in non-neuronal tissues (Maisonpierre, et al. 1990, Science 247: 1446-1451; Maisonpierre, et al. 1990, Neuron 5: 501-509), apparently because they serve as target-derived factors for axonal processes innervating these tissues (Thoenen, H.1991,Trends Neurosci. 14:165-170). The non-neuronal expression of Efl-1 and Efl-3 also raises the possibility that they serve as ligands for members of the Eph receptor family, such as Eck, that are expressed by non-neural cells.

Efl's described herein, peptide fragments thereof, or derivatives, including clustered ligands may be formulated in a suitable pharmacologic carrier to provide pharmaceutical compositions.

The Efl proteins, peptide fragments, or derivatives may be administered systemically or locally. Any appropriate mode of administration known in the art may be used, including, but not limited to, intravenous, intrathecal, intraarterial, intranasal, oral, subcutaneous, intraperitoneal, or by local injection or surgical implant. Sustained release formulations are also provided for.

### EXAMPLE 1: EXPRESSION CLONING OF EHK-1 BINDING LIGANDS

COS-7 cells were cultured In Dulbecco's modified Eagle's medium (DMEM) containing 10% fetal bovine serum (FBS), 1% each of penicillin and streptomycin (P/S) and 2 mM glutamine in an atmosphere of 5% CO₂. The human neuroblastoma cell line, SH-SY5Y (obtained from June Biedler, Sloan-Kettering) was cultured in Eagle's minimal essential medium (EMEM) with 10% FBS, (P/S) and 2 mM glutamine.

Full length human Efl cDNA clones were obtaining by screening a SHSY5Y cDNA library in the pJFE14 vector expressed in COS cells. Full length human Efl-4 cDNA clones were obtaining by screening a' human osteosarcoma 143B cell line cDNA library in the pJFE14 vector expressed in COS cells. The pJFE 14 vector, as shown in Figure 1, is a modified version of the vector pSR_{α} (Takebe, et al. 1988, Mol. Cell. Biol. 8: 466-472). The library was created using the two BSTX1 restriction sites In the pJFE14 vector.

COS-7 cells were transiently transfected with either the pJFE14 library or control vector by the DEAE-dextran transfection protocol. Briefly, COS-M5 cells were plated at a density of 1.0 x 10⁶ cells per 100 mm plate 24 hours prior to transfection. For transfection, the cells were cultured in serum-free DMEM containing 400 µg/ml of DEAE-dextran, 1 µM chloroquine, and 2 mM glutamine, and 1 µg of the appropriate DNA for 3-4 hours at 37°C in an atmosphere of 5% CO₂. The transfection media was aspirated and replaced with phosphate-buffered saline with 10% DMSO for 2-3 min. Following this DMSO "shock", the COS-7 cells were placed into DMEM with 10% FBS, 1% each of penicillin and streptomycin, and 2 mM glutamine for 48 hours.

The screening was conducted by direct localization of surface staining using an Ehk-1 receptorbody, which consisted of the extracellular domain of Ehk-1 fused to the IgG1 constant region. This receptorbody was prepared as follows: The Fc portion of human IgG1, starting from the hinge region and extending to the carboxy terminus of the molecule, was cloned from placental cDNA using PCR with oligonucleotides corresponding to the published sequence of human IgG1. Convenient restriction sites were also incorporated into the oligonucleotides so as to allow cloning of the PCR fragment into an expression vector. Expression vectors containing full length receptors were modified either by restriction enzyme digests or by PCR strategies so as to replace the transmembrane and intracellular domains with restriction sites that allow cloning the human IgG1 fragment into these sites; this was done in such a way as to generate a fusion protein with the receptor ectodomain as its amino-terminus and the Fc portion of human IgG1 as its carboxy-terminus. An alternative method of preparing receptorbodies is described in Goodwin, et. al. 1993, Cell 73:447-456).

Briefly, a 100 mm dish of COS cells was transfected with 1µg of SHSY5Y library plasmid DNA. Two days after transfection, the cells were probed by incubating them for 30 min with Ehk-1IgG (in the form of COS cell supernatants). The cells were then washed twice with PBS,fixed with methanol, and then Incubated for an additional 30 min with PBS/10% Bovine Calf Serumlanti-human IgG-alkaline phosphatase conjugate. After three PBS washes, cells were incubated In alk-phos substrate for 30-60 min. The dish was then Inspected microscopically for the presence of surface-stained cells. For each stained cell a small area surrounding it was scraped from the dish using a plastic pipette tip and plasmid DNA was then rescued and used to electroporate bacterial cells. Plasmid DNA prepared from cultures derived from these electroporations was used to transfect COS cells for a second round of enrichment. The second round was performed with standard panning techniques. After the second round of enrichment, single bacterial colonies were picked and plasmid DNA prepared from these colonies was tested for its ability to induce Ehk-1 staining in COS cells transfected with them.

Three clones from the SY5Y library were identified that exhibited binding to the Ehk-1 receptorbody. The first was identified as B61. The second and third clone, which are identical, and designated herein as Efl-2, appear to be different, but related to B61, as determined using partial nucleotide sequencing, suggesting that these may be family members of B61. All three ligands appear to be membrane bound. Further, like B61, Efl-2 is GPI-linked.

The human osteosarcoma cell line derived library resulted in a clone that encodes Efi-4. This ligand binds the Ehk-1, Ehk-2 and Ehk-3 and Eck receptors, but not the Elk receptor.

### EXAMPLE 2: CLONING OF EFL's

Regions of homology between B61 and Efl-2 can be used to identify additional EfI's as described in United States Patent Application Serial No. 08/144,992. For example, Figure 2 reveals two such regions of sequence homology that respectively bear the single letter amino acid sequences, V(F/Y)WNSSN (SEQ ID NO: 5) and NDY(V/L)DI(I/Y)CPHY (SEQ ID NO: 6), where letters in parenthesis indicate residues that differ in human and rat B61 as compared to the deduced Efl-2 protein. Degenerate oligodeoxynucleotides corresponding to these conserved protein regions can then be designed and used to prime PCR reactions using cDNAs made from various tissues, including embryonic and adult brain. Resulting amplified DNA fragments can then be cloned by insertion into plasmids, subjected to DNA sequencing and these sequences may then be compared with those of the known Efl's.

### EXAMPLE 3: EXPRESSION CLONING OF ELK BINDING LIGAND

COS-7 cells were cultured in Dulbecco's modified Eagle's medium (DMEM) containing 10% fetal bovine serum (FBS), 1% each of penicillin and streptomycin (P/S) and 2 mM glutamine in an atmosphere of 5% CO₂. The human neuroepithelioma line CHP100 (obtained from) was cultured in Eagle's minimal essential medium (EMEM) with 10% FBS, (P/S) and 2 mM glutamine.

Full length human Efl cDNA clones were obtaining by screening a CHP100 cDNA library in the pJFE14 vector expressed in COS cells. This vector, as shown in Figure 1, is a modified version of the vector pSR_{α} (Takebe, et al. 1988, Mol. Cell. Biol. 8:466-472). The library was created using the two BSTX1 restriction sites in the pJFE14 vector.

COS-7 cells were transiently transfected with either the pJFE14 library or control vector by the DEAE-dextran transfection protocol. Briefly, COS-M5 cells were plated at a density of 1.0 x 10⁶ cells per 100 mm plate 24 hours prior to transfection. For transfection, the cells were cultured in serum-free DMEM containing 400 µg/ml of DEAE-dextran, 1 µM chloroquine, and 2 mM glutamine, and 1 µg of the appropriate DNA for 3-4 hours at 37°C in an atmosphere of 5% CO₂. The transfection media was aspirated and replaced with phosphate-buffered saline with 10% DMSO for 2-3 min. Following this DMSO "shock", the COS-7 cells were placed into DMEM with 10% FBS, 1 % each of penicillin and streptomycin, and 2 mM glutamine for 48 hours.

The screening was conducted by direct localization of surface staining using an Elk receptorbody, which consisted of the extracellular domain of Elk fused to the IgG1 constant region. This receptorbody was prepared as follows: The Fc portion of human IgG1, starting from the hinge region and extending to the carboxy terminus of the molecule, was cloned from placental cDNA using PCR with oligonucleotides corresponding to the published sequence of human IgG1. Convenient restriction sites were also incorporated into the oligonucleotides so as to allow cloning of the PCR fragment into an expression vector. Expression vectors containing full length receptors were modified either by restriction enzyme digests or by PCR strategies so as to replace the transmembrane and intracellular domains with restriction sites that allow cloning the human IgG1 fragment into these sites; this was done in such a way as to generate a fusion protein with the receptor ectodomain as its amino-terminus and the Fc portion of human IgG1 as its carboxy-terminus. An alternative method of preparing receptorbodies is described in Goodwin, et. al. 1993, Cell 73:447-456).

Briefly, a 100 mm dish of COS cells was transfected with 1µg of CHP100 library plasmid DNA. Two days after transfection, the cells were probed by incubating them for 30 min with Elk-IgG (in the form of COS cell supernatants). The cells were then washed twice with PBS, fixed with methanol, and then incubated for an additional 30 min with PBS/10% Bovine Calf Serum/anti-human IgG-alkaline phosphatase conjugate. After three PBS washes, cells were incubated in alk-phos substrate for 30-60 min. The dish was then Inspected microscopically for the presence of surface-stained cells. For each stained cell a small area surrounding it was scraped from the dish using a plastic pipette tip and plasmid DNA was then rescued and used to electroporate bacterial cells. Plasmid DNA prepared from cultures derived from these electroporations was used to transfect COS cells for a second round of enrichment. The second round was performed with standard panning techniques. After the second round of enrichment, single bacterial colonies were picked and plasmid DNA prepared from these colonies was tested for its ability to induce Elk staining in COS cells transfected with them.

A clone identified that exhibited binding to the Elk receptorbody was deposited with the ATCC on April 12, 1994 and designated as 75734.

### EXAMPLE 4: ACTIVATION OF SOLUBLE EFL'S

Assays of membrane bound Efl's were conducted by growing either COS cells transfected with vector alone (COS-Mock) or COS cells transfected with Efl-3 expression vector, detaching the cells from the dishes with PBS + 1mM EDTA, pelleting, and resuspending in PBS. The cells were layered on top of reporter cell lines.

Soluble Efl-3 (Elk-1) and Efl-1 (B61 each tagged using the myc epitope at their C-termini [Stahl, et al. Science 263, 92-95 (1994)], were produced as COS cell supernatants and used as unclustered ligands or ligands clustered by antibodies. Ligand concentrations were estimated by quantitating the amount of myc epitope on slot blots; ligand clustering was accomplished by adding anti-myc monoclonal antibodies and anti-mouse polyclonal antibodies and incubating at 37°C for 1 hour before stimulating cells. Reporter cell lines were 3T3 fibroblasts transfected with Elk (Figure 5A and 5B) or C2C12 cells transfected with Ehk-1 (Figure 5C). Reporter cells on 10 cm dishes were starved for 4-6 hours in serum-free medium and then stimulated for 15 minutes as noted above, then solubilized and immunoprecipitated with antibodies recognizing the receptors. Immunoprecipitates were immunoblotted with antibodies to phosphotyrosine (Figure 5, UPPER PANELS), and subsequently stripped and reprobed with antibodies recognizing the receptors to visualize total receptor protein (Figure 5, LOWER PANELS).

To measure induction of growth responses in BAF cells mediated by Eck receptor chimera, stimulations were made with soluble EHk1L (Efl-2) and B61 (Efl-1), produced in COS cells with or without subsequent clustering. After two days of stimulation the number of viable cells was assessed by adding the vital dye MTT (3-{4,5-dimethylthiazol-2-yl}-2,5-diphenyltetrazolium bromide) for 4 hours, followed by solubilization and determination of optical density (O.D.), as previously described [Ip, et al., 1993, Neuron 10: 137-149) (Figure 8). The data described in Figure 9 results from MTT assays performed as above with soluble Ehk-1 L-Fc and B61-Fc produced in COS cells with and without subsequent clustering with anti-Fc antibodies. Because the catalytic domain of the Eck receptor does not mediate growth responses in BAF cells, a receptor chimera (in which the ectodomain of the Eck receptor was fused to the cytoplasmic domain of the FGF receptor) was instead introduced into BAF cells to make a reporter cell line.

As shown in Figure 5B, lanes 2 and 3, and Figure 5C, lane 2, no ligand-induced receptor activation, as judged by receptor phosphorylation, was observed using soluble forms of Elk-1 (Efl-3) or B61 (Efl-1) However, receptor phosphorylation was markedly induced by stimulating receptor expressing reporter cells with COS cells overexpressing membrane-bound forms of these ligands (eg. Fig.5A, lanes 1 and 2). To explain this discrepancy, we speculated that membrane attachment facilitates ligand clustering, which in turn promotes receptor multimerization and activation. To mimic ligand clustering in solution, secreted forms of the ligands were constructed with epitope tags added at their C-termini; antibodies against the tags were then used to aggregate the ligands. This type of clustering enabled previously inactive soluble ligands to strongly induce receptor tyrosine phosphorylation in reporter cells expressing Elk and Ehk-1 receptors (Fig.5B, lanes 1-5. and Fig. 5C, lanes 1-3). Similarly, as shown in Figure 7, with respect to Efl-3 (ELK-L), dimerization of the ligand is insufficient to induce biological active, whereas multimers had significant activity. These results demonstrate the need, as described herein, to assess the efficacy of a particular mechanism of clustering insofar as each particular ligand is concerned.

Such efficacy of the clustering may be determined by measuring phosphorylation of the receptor. Alternatively, proliferation in reporter cells expressing an appropriate receptor or receptor chimera, such as the Eck receptor chimera utilized in experiments the results of which are shown in Figure 6.

Dose-response studies demonstrated that clustering resulted in at least 100-fold greater potency, and saturable responses with low concentrations of ligand (comparable to those seen with ligands for other receptor families [Ip, et al. Neuron 10:137-149 (1993)], in both the phosphorylation assay (tested for the Elk ligand, data not shown) and in the proliferation assay (tested for Efl-1 and B61, Fig. 6). The small effect of unclustered B61 (Fig. 6B) is reminiscent of the recent finding that high concentrations of soluble B61 could induce Eck tyrosine phosphorylation [Bartley, et al. Nature 368: 558-560 (1994); in the latter study, saturation was not achieved even at 1-2 mg/ml concentrations of B61. These effects might be attributed to a weak ability of the soluble ligand itself to dimerize or aggregate, either spontaneously or as a result of purification. Soluble ligands are thought to activate their receptors by virtue of being bivalent or multivalent [Schlessinger, J. & Ullrich, A. Neuron 9:383-391 (1992)]; some ligands are monomers that contain two distinct receptor binding sites, while others achieve bivalency as covalent or non-covalently linked dimers. Our results define a new class of ligands that seem ineffective as soluble ligands, possibly because they are monovalent, although they can be artificially activated in solution by clustering. These ligands seem to depend on their membrane attachment to aggregate and activate their receptors. By limiting diffusion to two dimensions, membrane attachment could simply increase the likelihood of ligand-ligand collisions and thereby promote ligand dimerization, or more extensive ligand clustering, among ligands that interact only weakly in solution. Alternatively, specific mechanisms may exist to create cell-surface ligand clusters. In addition, ligand-receptor pairs would be expected to accumulate in areas of contact between cells bearing ligands and cells bearing receptors.

Any or all of these lateral aggregation effects could contribute to receptor multimerization and activation. The observation that the EPH-related, neuronally-expressed NUK receptor transiently concentrates in areas of cell-to-cell contact [Henkemeyer, et al. Oncogene 9:1001-1014 (1994)], supports the possibility that interactions between ligand bound to one membrane and receptor bound to another membrane could promote lateral aggregation.

### DEPOSIT OF MICROORGANISMS

The following microorganisms have been deposited with the American Type Culture Collection, 12301 Parklawn Drive, Rockville, Maryland 20852 in accordance with the Budapest Treaty.

| | ACCESSION NUMBER |
|---|---|
| pJFE14 containing *efl-2* | 75728 |
| pJFE14 containing *efl-3* | 75734 |
| pJFE 14 encoding efl-4 | 75921 |

The present invention is not to be limited in scope by the specific embodiments described herein.

## Claims

1. A method of enhancing the biological activity of the extracellular domain of an eph family ligand comprising:
a) expressing the soluble domain of the ligand with an epitope tag;
b) exposing said tagged soluble domain to anti-tag antibodies to form a clustered eph family ligand,
wherein said biological activity is measured by phosphorylation of the tyrosine kinase domain of an eph family receptor.

2. A method according to claim 1 wherein said tagging is at the C-terminus of the ligand.

3. A method according to claim 2 wherein said epitope tag is c-myc or the Fc domain of an IgG.

4. A method of enhancing the biological activity of the soluble domain of an eph family ligand selected from Efl-1 (B61) comprising:
a) expressing the soluble domain of the ligand with the Fc domain of IgG; and
b) allowing Fc dimers to form,
wherein said biological activity is measured by phosphorylation of the tyrosine kinase domain of an eph family receptor.

5. A anti-epitope antibody treated preparation of clustered epitope-tagged soluble eph family ligand which is obtainable by the method of claim 1, 2 or 3, wherein the eph family ligand is Efl-1 (B61).

6. A preparation according to claim 5 wherein said epitope tag is at the C-terminus of the ligand.

7. A preparation according to claim 6 wherein said epitope is myc or the Fc domain of an IgG.

8. A biologically active, clustered soluble eph family ligand comprising (soluble Efl)ₙ, wherein the soluble Efl is the extracellular domain of Efl-1 (B61) and n is 2 or greater,
wherein said biological activity is measured by phosphorylation of the tyrosine kinase domain of an eph family receptor.

## Patentansprüche

1. Verfahren zur Verstärkung der biologischen Aktivität der extrazellulären Domäne eines Liganden der eph-Familie umfassend:
(a) Expression der löslichen Domäne des Liganden mit einem Markierungsepitop;
(b) Exponieren der markierten löslichen Domäne mit Anti-Markierungs-Antikörpern zur Bildung eines Clusters von Liganden der eph-Familie,
wobei die biologische Aktivität anhand der Phosphorylierung der Tyrosinkinasedomäne eines Rezeptors der eph-Familie gemessen wird.

2. Verfahren nach Anspruch 1, wobei die Markierung sich am C-Terminus des Liganden befindet.

3. Verfahren nach Anspruch 2, wobei das Markierungsepitop c-myc oder die Fc-Domäne eines IgGs ist.

4. Verfahren zur Verstärkung der biologischen Aktivität einer löslichen Domäne eines Liganden der eph-Familie, ausgewählt aus Efl-1 (B61), umfassend:
(a) Expression der löslichen Domäne des Liganden mit der Fc-Domäne eines IgGs; und
(b) Zulassen der Bildung von Fc-Dimeren,
wobei die biologische Aktivität anhand der Phosphorylierung der Tyrosinkinasedomäne eines Rezeptors der eph-Familie gemessen wird.

5. Präparat eines geclusterten, Epitop-markierten, löslichen Liganden der eph-Familie, behandelt mit einem Anti-Epitop-Antikörper, welches erhältlich ist nach dem Verfahren der Ansprüche 1, 2 oder 3, wobei der Ligand der eph-Familie Efl-1 (B61)ist.

6. Präparat nach Anspruch 5, wobei sich das Markierungsepitop am C-Terminus des Liganden befindet.

7. Präparat nach Anspruch 6, wobei das Epitop myc oder die Fc-Domäne eines IgGs ist.

8. Biologisch aktiver, geclusterter, löslicher, Ligand der eph-Familie, umfassend (lösliches Etl)ₙ, wobei das lösliche Efl die extrazelluläre Domäne von Efl-1 (B61) ist und n 2 oder größer ist, wobei, die biologische Aktivität anhand der Phosphorylierung der Tyrosinkinasedomäne eines Rezeptors der eph-Familie gemessen wird.

## Revendications

1. Procédé d'augmentation de l'activité biologique du domaine extracellulaire d'un ligand de la famille eph, comprenant :
a) l'expression du domaine soluble du ligand avec une étiquette d'épitope ;
b) l'exposition du domaine soluble étiqueté à des anticorps anti-étiquette, pour former un ligand de famille eph groupé,
ladite activité biologique étant mesurée par phosphorylation au niveau du domaine tyrosine kinase d'un récepteur de la famille eph.

2. Procédé selon la revendication 1, dans lequel ledit étiquetage est à l'extrémité C-terminale du ligand.

3. Procédé selon la revendication 2, dans lequel ladite étiquette d'épitope est c-myc ou le domaine Fc d'une IgG.

4. Procédé d'augmentation de l'activité biologique du domaine soluble d'un ligand de la famille eph consistant en Efl-1 (B61), comprenant :
a) l'expression du domaine soluble du ligand avec le domaine Fc d'IgG ; et
b) le fait de laisser se former des dimères de Fc,
ladite activité biologique étant mesurée par phosphorylation au niveau du domaine tyrosine kinase d'un récepteur de la famille eph.

5. Préparation de ligand de la famille eph soluble, étiqueté par épitope, groupé, traitée par un anticorps anti-épitope, qui peut être obtenue par le procédé de la revendication 1, 2 ou 3, le ligand de la famille eph étant Efl-1 (B61).

6. Préparation selon la revendication 5, dans laquelle ladite étiquette d'épitope est à l'extrémité C-terminale du ligand.

7. Préparation selon la revendication 6, dans laquelle ledit épitope est myc ou le domaine Fc d'une IgG.

8. Ligand de famille eph soluble, groupé, biologiquement actif, comprenant (Efl soluble)ₙ, dans lequel l'Efl soluble est le domaine extracellulaire de Efl-1 (B61) et n est égal ou supérieur à 2,
ladite activité biologique étant mesurée par phosphorylation au niveau du domaine tyrosine kinase d'un récepteur de la famille eph.
